# EUROPEAN PATENT SPECIFICATION

(11) **EP 4 017 506 B1**
(45) Date of publication and mention of the grant of the patent: **25.06.2025**
(21) Application number: 20853769.6
(22) Date of filing: 25.06.2020
(51) Int. Cl.: A61L 24/02, A61L 24/00

(54) **HEMOSTATIC DEVICE AND METHOD**
BLUTSTILLENDE VORRICHTUNG UND VERFAHREN
DISPOSITIF HÉMOSTATIQUE ET PROCÉDÉ ASSOCIÉ

(30) Priority: 19.08.2019 US 201962888572 P
(43) Date of publication of application: 29.06.2022
(73) Proprietor: Joy Medical Devices Corporation, Kaohsiung City (TW)
(72) Inventor: JU, Chien-Ping, Kansas City, MO 64137 (US); LIN, Jiin-Huey Chern, Winnetka, IL 60093 (US)
(74) Representative: Grünecker Patent- und Rechtsanwälte PartG mbB
(86) International application number: PCT/US2020/039515
(87) International publication number: WO 2021/034406

(56) References cited:
- WO-A2-03/024316
- JP-A- 2018 168 098
- US-A1- 2005 170 012
- US-A1- 2007 021 703
- US-A1- 2008 063 671
- US-A1- 2014 275 287
- US-A1- 2017 106 119

## Description

### Field of the Invention

The present invention is related to a synthetic hemostatic agent, and in particular related to a synthetic hemostatic agent for reducing bone bleeding.

### Background of the Invention

Bone tissue is one of the most frequently transplanted tissues, second only to blood. A wide range of orthopedic, cardiothoracic, neurological, and maxillofacial procedures requires that the bone be cut or resected, either to operate on the osseous tissue itself or to gain access to other organs. Like any living tissue, bone bleeds when cut or fractured. To reduce the risk of postoperative complications, bone bleeding needs to be managed during surgery. Uncontrolled continuous bleeding can obscure the surgical field, prolong operating time, increase the risk of physiologic complications, and expose the patient to additional problems associated with blood transfusion. For example, for patients undergoing elective coronary artery bypass graft surgery, allogeneic blood transfusion has been shown to double the risk of infection *(Rogers et al., 2009).*

The hematopoietic elements are contained within a honeycombed network of vascular channels and can be a source of profuse bleeding. Electrocautery, which is useful in soft tissue hemostasis, functions primarily to collapse and seal blood vessels. To be effective in controlling bone bleeding, it would have to generate sufficient heat to create a coagulum that would physically block vascular channels. However, the thermal injury to the adjacent bone would be of such magnitude that any hemostatic benefit would be offset by the resultant bone necrosis.

Bone wax, a beeswax-based blend, sticks to bone blocking the vascular channels, providing immediate bone hemostasis. Although inexpensive and easy to use, bone wax has a number of troublesome side effects. Once applied to bone, bone wax remains at the site indefinitely. Bone wax is known to interfere with bone healing, elicit chronic inflammatory reactions, and increase infection rates (*Chun et al., 1988; Finn et al., 1992; Johnson and Fromm, 1981; Nelson et al., 1990; Sawan et al., 2010*)*.*

Products made of water-soluble alkylene oxide copolymers (AOC), such as Ostene^{®} (Baxter International, Inc.), were reported to provide immediate hemostasis yet remain at the site for only a few days. Research in animal models showed that AOC-based products did not increase infection rate or interfere with bone healing (*Sawan et al., 2010; Wellisz et al., 2006*)*.*

Bleeding from bone can also be controlled with soft tissue hemostats which can aid in the activation of the body's natural clotting mechanism and resorb from days to months. Examples of soft tissue hemostats include gelatin, thrombin, oxidized regenerated cellulose (ORC), collagen, platelet rich plasma (PRP) and fibrin sealants. All these soft tissue hemostats have their respective advantages and disadvantages. For example, gelatin products, if left in the wound, may act as a nidus for infection and abscess formation (*Lindstrom, 1956*)*.* Gelatin-based agents have also been reported to delay bone healing (*Schonauer et al., 2004*)*.* Thrombin works quickly and is easy to use. However, bovine thrombin has been known to trigger immunologic reactions *(Achneck et al., 2010).*

The low pH of ORC products was shown to have an antimicrobial effect. However, the acidity may cause inflammation of surrounding tissue and interfere with healing *(Tomizawa, 2005).* ORC has also been shown to promote a foreign body reaction and reduce bone repair in rat studies *(Ibarrola et al., 1985).* Microfibrillar collagen products derived from bovine corium, though shown promising in their ability to promote clotting in reconstructive spinal surgery *(Block, 2005),* were found related to infection and delayed bone healing. Inflammation and residual material upon resection at up to 90 days were found by *Barbolt et al. (2001).*

Fibrin sealants are made up of human thrombin and fibrinogen, that are combined at the time of surgery. Fibrin sealants are most frequently used for soft tissue hemostasis and often used as an adjunct to other hemostasis materials. PRP is derived from the patient's own blood by separating red blood cells from the fibrin and plasma in a centrifuge. The plasma, which forms a gel, is applied where hemostasis is needed. This technique relies on growth factors that trigger early wound healing said to be present in the PRP. The true benefit for PRP in bone hemostasis has yet to be confirmed *(Griffin et al., 2009),* and the technique has shown to be a markedly expensive bone hemostasis alternative.

US-A-2005/170012 discloses porous β-tricalcium phosphate granules for regeneration of bone tissue.

JP-A-2018168098 discloses a bone marrow hemostatic which has a hemostatic effect and facilitates bone regeneration on the hemostatic side.

### Summary of the Invention

The subject matter of the invention is as set out in the appended claims.

One aspect of the present invention relates to a novel use of a solid calcium compound in the fabrication of a hemostatic agent for reducing bleeding from a surgical site during and/or after a surgical treatment in a patient, wherein the solid calcium compound is selected from the group consisting of calcium phosphate, calcium sulfate, calcium carbonate, calcium oxide, calcium hydroxide, hydroxyapatite, and a combination thereof, wherein the solid calcium compound is in a form of porous granules having a granular size of about 0.5 mm to about 2.5 mm and comprising interconnected pores having a pore size in a range of about 30 mm to about 300 µm.

Preferably, the hemostatic composition further comprises a phosphorus source, a sulfur source, or a phosphorus source and a sulfur source.

Preferably, the solid calcium compound has a porous structure with a porosity of about 30 vol% to about 90 vol%, and more preferably about 60 vol% to about 80 vol%.

Preferably, the solid calcium compound is in a form of porous granules having a granular size of about 0.5 mm to about 1.5 mm.

Preferably, pores of the porous structure have a pore size in a range of 50 µm to about 250 µm.

Preferably, the surgical site comprises a bone cavity or a bone cut, and the bleeding is a bone bleeding, wherein the applying a hemostatic composition to a surgical site comprises contacting the bone cavity or the bone cut with the hemostatic composition.

Preferably, bone marrow is exposed by the bone cavity or the bone cut.

Preferably, the surgical treatment is a sternum closure treatment and the surgical site comprises a surgically-created sternum cavity.

Preferably, the phosphorus source comprises a phosphate, and the sulfur source comprises a sulfate.

Preferably, the solid calcium compound is calcium phosphate, calcium sulfate, or a mixture thereof, and more preferably the solid calcium compound is calcium phosphate.

Preferably, the calcium phosphate comprises tetracalcium phosphate (TTCP), dicalcium phosphate (DCP), tricalcium phosphate, monocalcium phosphate, hydroxyapatite, or a mixture thereof.

Preferably, the calcium phosphate comprises hydroxyapatite, tetracalcium phosphate and dicalcium phosphate.

Preferably, the solid calcium compound is a mixture of calcium phosphate and calcium sulfate.

Preferably, the calcium sulfate is calcium sulfate hemihydrate (CSH), calcium sulfate dihydrate (CSD), anhydrous calcium sulfate, or a mixture thereof. More preferably, the calcium sulfate is calcium sulfate hemihydrate (CSH), calcium sulfate dihydrate (CSD) or a mixture thereof.

### Detailed Description of the Invention

The present description discloses (but not limited to) the following aspects:
1. A hemostatic agent for reducing bleeding during and after a surgical treatment, wherein said hemostatic agent comprises a calcium source; wherein said hemostatic agent is in a porous form and has a porosity of about 40-90 vol%, preferably 60-80 vol%.
2. The composition of said hemostatic agent in (1) contains at least 10 at% inorganic calcium ions.
3. The calcium ions in (2) exist in an inorganic, biocompatible calcium compound comprising at least one selected from the group consisting of calcium phosphate, calcium sulfate, calcium carbonate, calcium oxide, and calcium hydroxide.
4. The composition of said hemostatic agent in (2) further comprising a phosphorus source and/or a sulfur source.
5. Said porous form in (1) is a porous granular form.
6. The granules of said porous granular form in (5) have a size of about 0.1- 2.5 mm and preferably about 0.5-1.5 mm.
7. The pores of said granules in (6) have a pore size in the range of about 30 to 300 µm and preferably 50 to 250 µm, and are preferably interconnected.
8. Said bleeding in (1) is a bone bleeding.
9. Said bone in (8) is a sternum.
10. A hemostatic treatment for sternum closure surgery comprising inserting a hemostatic agent into the bony cavity prior to closure of the surgically opened sternum; wherein said hemostatic agent is a synthetic, inorganic agent containing at least 10 at% inorganic calcium ions; wherein said calcium ions exist in any form of inorganic, biocompatible calcium compounds comprising at least one of the group consisting of calcium phosphate, calcium sulfate, calcium carbonate, calcium oxide, and calcium hydroxide; wherein said hemostatic agent is in a porous form and has a porosity of about 40-90 vol%, preferably 60-80 vol%.

Advantages of the present inventive hemostatic device and method for reducing bone bleeding during and post surgeries
(1) Unlike the existing organic and animal/human tissue-derived hemostatic devices which all have their aforementioned respective potential problems, the present inventive hemostatic agent is a 100% inorganic and entirely synthetic agent.
(2) The inventive device is designed to promote hemostasis during and post bone surgeries without potential organic and especially human or animal tissue-related safety risks. For this purpose, the composition of the present inventive device is designed to contain at least 10 at% inorganic calcium ions. The calcium ions in the product may exist in any form of inorganic, biocompatible calcium compounds such as calcium phosphate, calcium sulfate, calcium oxide, calcium hydroxide, etc.
(3) To enhance the hemostatic effect, the hemostatic agent may further comprise phosphorus and/or sulfur ions.
(4) For the purpose of minimally invasive delivery, the hemostatic agent is designed to have a granular form. The granules are designed to have a size of about 0.1- 2.5 mm and preferably about 0.5-1.5 mm.
(5) In order to maximize the exposure of the hemostatic agent and at the same time to avoid the disintegration of the porous structure during delivery, the granules of the hemostatic agent are designed to have a highly porous form with a porosity level of about 40-90 vol% and preferably about 50-80 vol%.
(6) To assist blood to penetrate into the interior of each granule, the pores of the granules are designed to have a pore size substantially in the range of 30 to 300 µm, preferably 50 to 250 µm, and to be substantially interconnected.

To the knowledge of the present inventors, there have not been any prior art hemostatic device having all these characteristics.

### Clinical trials

Two complicated cases of open heart surgeries were completed using the hemostatic agent of the present invention in National Cheng Kung University Hospital, Tainan, Taiwan. The doctors used EZECHBONE^{®} Granule to promote the union of median sternotomy of the patients. The doctors surprisingly found EZECHBONE^{®} Granule also possesses good efficacy on hemostasis of bone marrow. 1st case was a 49 y/o female who was a CKD case receiving regular hemodialysis. Osteoporosis is one major problem of her. She underwent CABG*4 due to recent myocardial infarction. 0.24g EZECHBONE^{®} Granule was used. 2nd case was a 74 y/o old man who was noted huge aortic aneurysm (maximal diameter was 7.1 cm) extending from ascending aorta, aortic arch, and proximal descending aorta. Total ascending aorta and arch were replaced by prosthetic graft. Proximal descending aorta was implanted with cover stent graft, i.e. frozen elephant trunk stenting. Besides, right and left coronary arteries were bypassed with saphenous vein grafts. Innominate, left carotid and left subclavian arteries were also revascularized with prosthetic grafts. After operation, two cases recovered smoothly. Also, EZECHBONE^{®} Granules showed wonderful hemostatic efficacy on them. For 1st case, 1st day after operation, only 80c.c. blood was noted from drainage tubes (greatly less than the average amount of HD open heart patient: 200~300c.c./day). Therefore, chest tubes were removed on the 2nd day after operation (also shorter than the average HD patients). For 2nd case, overall blood drainage was 170c.c. (pericardial tubes 110c.c. and left pleural chest tube: 60 c.c.). In this hospital, averaged POD-1 drainage amount≥ 800 c.c. The 2nd day: overall 170c.c. but pleural chest tube: 50c.c. So, left pleural chest tube was removed. The 3rd day: pericardial tubes 100c.c. and the doctors separated them into 2 individual tubes. The 4th day: 1st tube 70c.c. and the 2nd tube 20c.c. So the 2nd tube was removed. The 5th day: 1st tube <70c.c. and was removed. So, from these 2 cases experiences, the doctors found EZECHBONE^{®} Granule not only promotes well bone union, but also possesses good effectiveness on hemostasis.

EZECHBONE^{®} Granule is available from JOY MEDICAL DEVICES CORPORATION, 1F., No. 63, Luke 2nd Road, Luzhu District, Kaohsiung City, Taiwan. EZECHBONE^{®} Granule has a particle size of about 0.4-1.2 mm, and a porosity of about 70-80 vol%, and is TTCP/DCPA/CSH-derived porous granules prepared based on the method disclosed in US patent No. 8,784,551. The porous granules of calcium compound can be prepared from a mixed powers of TTCP/DCP/calcium sulfate (preferably TTCP/DCPA/CSH; more preferably TTCP/DCPA/CSH/CSD) and a pore forming agent, which is then mixed with a NH⁴⁺ solution to form a paste. The paste is hardened into a block in a mold, followed by washing the pore forming agent from the hardened block and breaking the block into porous granules; or breaking the block prior to washing. The porous granules are composed of hydroxyapatite, calcium sulfate (primarily CSD), and less significantly unreacted TTCP, DCP, and CSH. (DCPA: dicalcium phosphate anhydrous)

### References

Achneck HE, Sileshi B, Jamiolkowski RM, Albala DM, Shapiro ML, Lawson JH ", A comprehensive review of topical hemostatic agents: efficacy and recommendations for use", Ann Surg 251:217-28, 2010*.*

Barbolt TA, Odin M, Leger M, Kangas L, "Pre-clinical subdural tissue reaction and absorption study of absorbable hemostatic devices ", Neurol Res 23: 537-42, 2001*.*

Block JE, "Severe blood loss during spinal reconstructive procedures: the potential usefulness of topical hemostatic agents", Med Hypotheses 65(3): 617-21, 2005*.*

Chun PKC, Virmani R, Mason TE, Johnson F, "Bone wax granuloma causing saphenous vein thrombosis. " Am. Heart J. 115:1310-1313, 1988*.*

Finn MD, Schow SR, Scneiderman ED, "Osseous regeneration in the presence of four common hemostatic agents", J. Oral Maxillofac. Surg. 50:608-612, 1992*.*

Griffin XL, Smith CM, Costa ML. "The clinical use of platelet-rich plasma in the promotion of bone healing: a systematic review", Injury 40:158-62, 2009*.*

Ibarrola JL, Bjorenson JE, Austin BP, Gerstein H, "Osseous reactions to three hemostatic agents ", J Endod 11: 75-83, 1985*.*

Johnson P, Fromm D, "Effects of bone wax on bacterial clearance. " Surgery 89:206-209, 1981*.*

Lindstrom PA, "Complications from the use of absorbable hemostatic sponges. " AMA Arch Surg. Iss. 73: 133-141, 1956*.*

Nelson DR, Buxton TB, Luu QN, Rissing JP, "The promotional effect of bone wax on experimental Staphylococcus aureus osteomyelitis ", J Thorac Cardiovasc Surg 99: 977-80, 1990*.*

Rogers M, Blumberg N, Saint S, Langa KM, Nallamothu BK, "Hospital variation in transfusion and infection after cardiac surgery: a cohort study", BMC Medicine 7:37doi:10.1186/1741-7015-7-37, 2009*.*

Sawan A, Elhawary Y, Zaghlool AM, Abdel RM, "Controversial role of two different local haemostatic agents on bone healing. " Journal of American Science 6(12): 155-163, 2010*.*

Schonauer C, Tessitore E, Barbagallo G, Albanese V,Moraci A, "The use of local agents: bone wax, gelatin, collagen, oxidized cellulose. " Eur Spine J. 13 (suppl 1): S89-S96, 2004*.*

Tomizawa Y, "Clinical benefits and risk analysis of topical hemostats: a review. " J Artif Organs 8: 137-42, 2005*.*

Wellisz T, Armstrong JK, Cambridge J, Fisher TC, "Ostene, a new water-soluble bone hemostasis agent. " J Craniofac Surg 17: 420-425, 2006*.*

## Claims

1. Use of a solid calcium compound in the fabrication of a hemostatic agent for reducing bleeding from a surgical site during and/or after a surgical treatment in a patient, wherein the solid calcium compound is selected from the group consisting of calcium phosphate, calcium sulfate, calcium carbonate, calcium oxide, calcium hydroxide, hydroxyapatite, and a combination thereof, wherein the solid calcium compound is in a form of porous granules having a granular size of about 0.5 mm to about 2.5 mm and comprising interconnected pores having a pore size in a range of about 30 µm to about 300 µm.

2. The use of claim 1, wherein the hemostatic agent further comprises a phosphorus source, a sulfur source, or a phosphorus source and a sulfur source.

3. The use of claim 1, wherein the solid porous granules has a porosity of about 30 vol% to about 90 vol%.

4. The use of claim 3, wherein the porosity is of about 60 vol% to about 80 vol%.

5. The use of claim 1, wherein the granular size is of about 0.5 mm to about 1.5 mm.

6. The use of claim 1, wherein the surgical site comprises a bone cavity or a bone cut, and the bleeding is a bone bleeding, wherein the hemostatic agent is for contacting the bone cavity or the bone cut to reduce the bleeding.

7. The use of claim 6, wherein bone marrow is exposed by the bone cavity or the bone cut.

8. The use of claim 1, wherein the surgical treatment is a sternum closure treatment and the surgical site comprises a surgically-created sternum cavity.

9. The use of claim 1, wherein the solid calcium compound is calcium phosphate, calcium sulfate, hydroxyapatite or a mixture thereof.

10. The use of claim 1, wherein the solid calcium compound is a mixture of hydroxyapatite, calcium phosphate and calcium sulfate.

11. The use of claim 10, wherein the calcium phosphate is tetracalcium phosphate (TTCP), dicalcium phosphate, monocalcium phosphate or a mixture thereof; and the calcium sulfate is calcium sulfate hemihydrate (CSH), calcium sulfate dihydrate (CSD), anhydrous calcium sulfate, or a mixture thereof.

12. The use of claim 11, wherein the calcium phosphate is tetracalcium phosphate (TTCP), dicalcium phosphate, or a mixture thereof; and the calcium sulfate is calcium sulfate hemihydrate (CSH), calcium sulfate dihydrate (CSD), or a mixture thereof.

## Patentansprüche

1. Verwendung einer festen Calciumverbindung bei der Herstellung eines hämostatischen Mittels zur Verringerung von Blutungen aus einer chirurgischen Stelle während und/oder nach einer chirurgischen Behandlung bei einem Patienten, wobei die feste Calciumverbindung aus der Gruppe ausgewählt ist, die aus Calciumphosphat, Calciumsulfat, Calciumcarbonat, Calciumoxid, Calciumhydroxid, Hydroxyapatit und einer Kombination davon besteht, wobei die feste Calciumverbindung in Form von porösen Körnchen vorliegt, die eine Korngröße von etwa 0,5 mm bis etwa 2,5 mm aufweist und miteinander verbundene Poren mit einer Porengröße in einem Bereich von etwa 30 µm bis etwa 300 µm umfasst.

2. Verwendung nach Anspruch 1, wobei das hämostatische Mittel ferner eine Phosphorquelle, eine Schwefelquelle oder eine Phosphorquelle und eine Schwefelquelle umfasst.

3. Verwendung nach Anspruch 1, wobei das feste poröse Granulat eine Porosität von etwa 30 bis etwa 90 Vol.-% aufweist.

4. Verwendung nach Anspruch 3, wobei die Porosität etwa 60 Vol.-% bis etwa 80 Vol.-% beträgt.

5. Verwendung nach Anspruch 1, wobei die Granulatgröße etwa 0,5 mm bis etwa 1,5 mm beträgt.

6. Verwendung nach Anspruch 1, wobei die Operationsstelle eine Knochenhöhle oder einen Knochenschnitt umfasst und die Blutung eine Knochenblutung ist, wobei das hämostatische Mittel dazu dient, die Knochenhöhle oder den Knochenschnitt zu kontaktieren, um die Blutung zu reduzieren.

7. Verwendung nach Anspruch 6, wobei das Knochenmark durch die Knochenhöhle oder den Knochenschnitt freigelegt wird.

8. Verwendung nach Anspruch 1, wobei es sich bei der chirurgischen Behandlung um eine Brustbeinverschlussbehandlung handelt und die Operationsstelle eine chirurgisch geschaffene Brustbeinhöhle umfasst.

9. Verwendung nach Anspruch 1, wobei es sich bei der festen Calciumverbindung um Calciumphosphat, Calciumsulfat, Hydroxyapatit oder eine Mischung davon handelt.

10. Verwendung nach Anspruch 1, wobei die feste Calciumverbindung eine Mischung aus Hydroxylapatit, Calciumphosphat und Calciumsulfat ist.

11. Verwendung nach Anspruch 10, wobei das Calciumphosphat Tetracalciumphosphat (TTCP), Dicalciumphosphat, Monocalciumphosphat oder ein Gemisch davon ist; und das Calciumsulfat Calciumsulfat-Halbhydrat (CSH), Calciumsulfat-Dihydrat (CSD), wasserfreies Calciumsulfat oder ein Gemisch davon ist.

12. Verwendung nach Anspruch 11, wobei das Calciumphosphat Tetracalciumphosphat (TTCP), Dicalciumphosphat oder ein Gemisch davon ist; und das Calciumsulfat Calciumsulfat-Halbhydrat (CSH), Calciumsulfat-Dihydrat (CSD) oder ein Gemisch davon ist.

## Revendications

1. Utilisation d'un composé de calcium solide dans la fabrication d'un agent hémostatique pour réduire un saignement provenant d'un site chirurgical pendant et/ou après un traitement chirurgical chez un patient, dans laquelle le composé de calcium solide est choisi dans le groupe constitué de phosphate de calcium, sulfate de calcium, carbonate de calcium, oxyde de calcium, hydroxyde de calcium, hydroxyapatite, et d'une combinaison de ceux-ci, dans laquelle le composé de calcium solide se présente sous la forme de granules poreux ayant une taille granulaire d'environ 0,5 mm à environ 2,5 mm et comprenant des pores interconnectés ayant une taille de pore dans une plage d'environ 30 µm à environ 300 µm.

2. Utilisation selon la revendication 1, dans laquelle l'agent hémostatique comprend en outre une source de phosphore, une source de soufre, ou une source de phosphore et une source de soufre.

3. Utilisation selon la revendication 1, dans laquelle les granules poreux solides ont une porosité d'environ 30 % en volume à environ 90 % en volume.

4. Utilisation selon la revendication 3, dans laquelle la porosité est d'environ 60 % en volume à environ 80 % en volume.

5. Utilisation selon la revendication 1, dans laquelle la taille granulaire est d'environ 0,5 mm à environ 1,5 mm.

6. Utilisation selon la revendication 1, dans laquelle le site chirurgical comprend une cavité osseuse ou une coupure osseuse, et le saignement est un saignement osseux, dans laquelle l'agent hémostatique est destiné à entrer en contact avec la cavité osseuse ou la coupure osseuse pour réduire le saignement.

7. Utilisation selon la revendication 6, dans laquelle la moelle osseuse est exposée par la cavité osseuse ou la coupure osseuse.

8. Utilisation selon la revendication 1, dans laquelle le traitement chirurgical est un traitement de fermeture du sternum et le site chirurgical comprend une cavité de sternum créée par chirurgie.

9. Utilisation selon la revendication 1, dans laquelle le composé de calcium solide est du phosphate de calcium, du sulfate de calcium, de l'hydroxyapatite ou un mélange de ceux-ci.

10. Utilisation selon la revendication 1, dans laquelle le composé de calcium solide est un mélange d'hydroxyapatite, de phosphate de calcium et de sulfate de calcium.

11. Utilisation selon la revendication 10, dans laquelle le phosphate de calcium est du phosphate tétracalcique (TTCP), phosphate dicalcique, phosphate monocalcique ou un mélange de ceux-ci ; et le sulfate de calcium est du sulfate de calcium hémi-hydraté (CSH), sulfate de calcium di-hydraté (CSD), sulfate de calcium anhydre, ou un mélange de ceux-ci.

12. Utilisation selon la revendication 11, dans laquelle le phosphate de calcium est du phosphate tétracalcique (TTCP), phosphate dicalcique ou un mélange de ceux-ci ; et le sulfate de calcium est du sulfate de calcium hémi-hydraté (CSH), sulfate de calcium di-hydraté (CSD), ou un mélange de ceux-ci.
